# EUROPEAN PATENT APPLICATION

(11) **EP 3 971 549 A1**
(43) Date of publication of application: **23.03.2022**
(21) Application number: 20197369.0
(22) Date of filing: 22.09.2020
(51) Int. Cl.: G01N 1/24, G01N 1/08, G01N 1/22, A61B 10/02

(54) **METHOD, TOOL AND APPARATUS FOR DISSECTING AND TRANSFERRING BIOLOGICAL MATERIAL**

(71) Applicant: Xyall B.V., 5633 AJ Eindhoven (NL)
(72) Inventor: THIJSSEN, Edwin Johannes Richardus Wilhelmus, 5441 XB Oeffelt (NL); HAAZEN, Nicole Catharina Elisabeth, 5685 JW Best (NL); LEMBRECHTS, Thomas Patrick Anne-Lise, 5617 AK Eindhoven (NL); VIEHMANN, Kelsey, 6003 AD Weert (NL); WIMBERGER-FRIEDL, Reinhold, 5633 AJ Eindhoven (NL)
(74) Representative: DeltaPatents B.V.

(57) **Abstract**

In one aspect, the invention relates to a method of transferring biological material (117) from a sample disposed on a planar substrate (115) into a collection tube, using a dissection tool (120) that has an internal passageway (121) extending between first and second ends of the tool and an air-permeable filter element (125) that spans the internal passageway. The tool first end is provided with an orifice (123) and a scraping blade (124). The method comprising steps of:
a) physically detaching the biological material from the planar substrate (115) using the scraping blade (124) of the dissection tool;
b) during step a), generating an underpressure at the tool second end by connecting the internal passageway (121) to a vacuum generator (150), to create an uplifting airstream at the tool orifice (123), which suctions detached material into the internal passageway (121), where it is held at an underside of the filter element (125);
c) attaching the collection tube to an outer surface of the dissection tool in an airtight manner; and
d) generating an overpressure at the tool second end, by connecting the internal passageway to a pressure reservoir (160), thereby creating a pressure pulse that ejects the biological material held at the underside of the filter element, transferring the material (117) into the collection tube.

## Description

### FIELD OF THE INVENTION

The invention relates to a method of dissecting biological material disposed on a planar substrate, such as a glass slide, and transferring the dissected material to a receptacle for further analysis. The invention further relates to an associated apparatus and to a disposable tool for use in the apparatus.

### BACKGROUND ART

To perform a molecular analysis of a tumor, for the purposes of oncology diagnostics, a certain amount and concentration of tumor cells must be present in the sample to be analyzed. Tumor tissue is heterogenous and contains other tissue and cell types. Therefore, a region of interest (ROI) is typically defined as the sample to be dissected from a thin section of tissue disposed on a microscope slide. Manual methods of dissection are most common, in which a lab technician scrapes material from the ROI using e.g. a scalpel blade and transfers the scraped material into a collection tube. This requires a great deal of skill and even when performed by a highly skilled technician, there is no guarantee that only material from the ROI will end up in the collection tube.

An example of an automated device for extracting material from a biological sample is disclosed in WO 2012/102779. The device comprises an extraction tool, which has a rotating cutting tip for disrupting material from a region of the sample and a liquid dispensing port and a liquid aspiration port, located in close proximity to the cutting tip. The device is configured such that liquid is dispensed at the cutting tip and such that disrupted material and dispensed liquid are aspirated via the aspiration port into the extraction device. When liquid is used in the extraction of the disrupted material, the liquid becomes part of the sample and may thus dilute the amount and/or concentration of material, leading to variations in yield in the downstream process.

It is also known to use laser capture microdissection (LCM) to isolate a concentrated population of individual cells or precise anatomical regions of tissue from tissue sections on a microscope slide. A method and device for transferring a microscopic, isolated sample, particularly a membrane-supported micro-dissected specimen, from an object table to an analysis arrangement, is disclosed in US 8573073. The device is equipped with nano-suction means and comprises a suction tube with a terminal membrane and a vacuum/overpressure unit coupled to the suction tube for sucking or blowing the sample onto or from the terminal membrane.

There is still room for improvement in terms of defining a more straightforward method and apparatus for automated dissection of biological material that allows the material to be collected and transferred for analysis in an efficient and reliable manner with minimal contamination.

### SUMMARY OF THE INVENTION

In a first aspect, the invention relates to a method of dissecting and transferring biological material from a sample disposed on a planar substrate, such as a glass slide, into a collection tube, using a dissection tool that has an internal passageway extending between first and second ends of the tool and an air-permeable filter element that spans the internal passageway. The tool first end is provided with an orifice and a scraping blade at one side of the orifice. The method comprises steps of:
a) physically detaching the biological material from the planar substrate using the scraping blade of the dissection tool;
b) during step a), generating an underpressure at the second end of the dissection tool, to create an uplifting airstream at the tool orifice, which suctions detached material into the internal passageway, where it is held at an underside of the filter element;
c) arranging the collection tube around the orifice, so as to be in sealing contact with a connection interface on the dissection tool; and
d) generating an overpressure at the tool second end, so as to create an airstream and pressure pulse that ejects the biological material held at the underside of the filter element, transferring the material into the container tube.

The method of the invention enables a "dry" method of transferring the biological material from the glass slide straight to the collection tube, thereby minimizing the risk of contamination. Furthermore, a single tool can be used to dissect, collect and transfer the biological material, which not only simplifies the construction of the equipment that is needed to implement the method, but also further reduces the risk of contamination. The method does not utilize any liquids which would dilute the biological material and impact the methods and quality of the molecular analysis of the collected biological material.

The step of detachment comprises bringing the scraping blade into contact with the planar substrate and moving the dissection tool relative thereto. For simplicity, the planar substrate will be referred to hereafter as a glass slide, although as will be understood, any suitable type of substrate on which biological samples are disposed may be used in the method of the invention.

Advantageously, the method comprises controlling the relative positions of the tool and the glass slide such that the scraping blade engages with the biological material only in a region of interest. This optimises the amount and purity of the material that is gathered for analysis.

The step of generating an underpressure suitably comprises connecting the internal passageway of the dissection tool, at the tool second end, to a vacuum generator and the step of generating an overpressure comprises connecting the internal passageway to a pressure reservoir.

Preferably, the internal passageway of the dissection tool is selectively connectable to the vacuum generator or the pressure reservoir via a valve and the method comprises switching the valve from a first position in which the internal passageway is in fluid communication with the vacuum generator to a second position in which the internal passageway is in fluid communication with the pressure reservoir.

The pressure differential between the pressure reservoir and the sealed collection tube creates the pressure pulse that ejects the biological material from the filter element into the collection tube.

In a preferred embodiment, the method comprises a further step of using the vacuum generator to at least partially evacuate the collection tube before the tool second end is connected to the pressure reservoir. The advantage of evacuating the collection tube is that this increases the pressure differential between the sealed collection tube and the pressure reservoir. A further advantage of evacuating the collection tube is that the atmosphere may serve as the pressure reservoir and the step of generating an overpressure may simply comprise exposing the evacuated tube to atmospheric pressure.

Preferably, the pressure reservoir supplies a pressure higher than atmospheric pressure, e.g. 200 - 400 kPa, to ensure that the generated pressure pulse is sufficient to eject the biological material, which may be sticky in nature and adhere to the underside of the filter element. The sealing contact between the collection tube and the connection interface on the dissection tool involves a certain clamping force. Preferably, the pressure reservoir supplies a pressure that is higher than the pressure in the sealed collection tube and lower than the clamping force between the collection tube and the tool.

In embodiments of the method where the step of generating an overpressure involves generating a pressure of greater than atmospheric pressure, the method suitably comprises a subsequent step of allowing the collection tube to equilibrate to atmospheric pressure. This can be achieved via controlled leakage of the pressure reservoir, or by switching the valve to a third position in which the internal passageway is in fluid communication with the atmosphere or other suitable method. The collection tube can then be removed from the dissection tool.

Suitably, the method comprises controlling the airflow during the step of dissection. In a further development, the method comprises a step of measuring pressure or measuring airflow downstream from the filter element of the dissection tool or a step of estimating an area of biological material that has been dissected from the glass slide. The airflow is controlled by adjusting the restriction of a variable restrictor in response to the measured pressure or measured airflow or estimated area of dissected material. The advantage of the further development will be described later with reference to the apparatus of the invention.

In a second aspect, the invention defines a dissection tool for use in the method. The tool has first and second ends, an internal passageway extending therebetween and an air-permeable filter element arranged in the internal passageway. The tool first end is provided with an orifice and a scraping blade. The scraping blade is preferably made of a metal having a high yield stress.

The filter element divides the internal passageway into first and second sections. The first section of the passageway will be defined as the section between the filter element and the tool orifice. The first section of the passageway is preferably straight and has a longitudinal centre axis L. The diameter of the passageway first section may be constant over its length or may vary. For example, the passageway may narrow towards the tool orifice. In use, the part of the dissection tool that surrounds the first section of the passageway serves as a suction nozzle and will be referred to as the tool nozzle.

The scraping blade is arranged at one side of the tool orifice. In one embodiment, the scraping blade is a separate part that extends from an end face of the nozzle. The blade may be embedded in the end face and/or attached via adhesive bonding or other suitable joining method. Alternatively, the nozzle may be formed from a polymer material that is overmoulded to the blade. Advantageously, the entire tool body may be formed from moulded polymer material. The moulded material can be an injection moulding grade thermoplastic polymer. The grade can be a glass- ceramic-, or carbon-filled material. The polymer can be from the class of polypropylene, polyester, polycarbonate, ABS, or other engineering thermoplastic material and blends of those that are commonly used in engineering and high precision applications.

The scraping blade has a scraping edge of width w that makes contact with the biological material on the glass slide. In some examples, the blade is a flat, chisel-like part. In other examples, the scraping blade is curved around a blade axis and comprises a thin-walled tube section. The tube section may be a continuous thin-walled cylinder or a partial cylinder such as a semi-cylinder. An end face of the tube section serves as the scraping edge. In one embodiment, the nozzle is at least partly formed from a thin-walled tube section and the scraping blade forms part of the nozzle. The tube section may have a wall thickness of 30 - 100 µm and an outer diameter of 3.0 - 15 mm. The scraping edge of the tube section or the scraping edge of a flat blade can be perpendicular to the blade axis or may be angled for optimized scraping performance. In some examples, the blade has a ground, sharpened edge.

Typically, a dissection tool according to the invention is arranged such that the scraping blade engages a horizontal surface of the glass slide at an angle α thereto, which will be defined as a scraping angle. In some embodiments, the scraping blade extends in a direction parallel to the longitudinal axis L of the nozzle and the scraping angle is defined by the orientation of the nozzle axis relative to the slide surface.

In other embodiments, particularly where the scraping blade is a separate metal part extending from an end face of the nozzle, the scraping blade has a blade axis that extends at an angle relative to the longitudinal axis L. The nozzle may thus be arranged such that the longitudinal axis L is perpendicular to the slide surface, and the scraping angle is defined by the angle between the slide surface and the blade axis. As will be understood, the scraping angle α can be varied by adjusting the angular orientation of the nozzle axis.

The tool second end is provided with a first connection interface for releasably connecting the dissection tool in an airtight manner to a mating interface on a tool carrier. The first connection interface may be a click fitting, a bayonet fitting, a clamp fitting or an internal screw thread. Preferably, the first mechanical interface is a quick coupling, to enable automated detachment of the tool when sufficient material has been collected and transferred and automated attachment of a new dissection tool.

The tool further comprises a second connection interface, at an outer surface of the tool body, for establishing a seal with the collection tube. In one example, the second connection comprises a collar with an outer diameter that is dimensioned to mate with the internal diameter of a collection tube. Preferably, the collar comprises a larger-diameter flange part for contacting an upper rim of the collection tube when it is placed around the nozzle. The seal may then be formed between the upper rim and the flange when the internal diameter of the collection tube is larger than the diameter of the collar, whereby the collection tube is pressed against the flange with a certain force to achieve an airtight connection.

In one embodiment, the collar is a separate part formed of elastomeric material that is mounted around the tool body. Examples of suitable materials include rubbers (cross-linked, olefins, urethanes or silicones) and thermoplastic elastomers, such as polyesters, polyurethanes, polyolefins. The collar may have outer diameter that is slightly larger than the internal diameter of a standard-sized collection tube.

In a further example, particularly where the tool body is at least partly formed in a moulding process, a moulded outer surface of the tool body is suitably dimensioned to serve as the connection interface for the collection tube.

The tool of the invention further comprises an air-permeable filter element arranged within the internal passageway and spanning the full diameter thereof. Suitably, the filter element is made of a porous material with a pore size that does not allow passage of the dissected material, e.g. 10, 50, 100 or 200 µm, depending on the sample from which material is dissected. The filter element may have a woven or non-woven structure and is preferably made of an apolar synthetic material such as polyethylene, polypropylene, polyvinylchloride, polyvinylidenefluoride or Teflon. The filter element may have a diameter of between 3.0 and 15.0 mm. Alternatively, the filter may be a thin substrate with an array of defined pores made by lithography, etching or laser ablation.

In a further development, the scraping blade of the dissection tool is provided with an anti-stick coating such as NiF or perfluoralkane, to prevent sticking of the dissected material and to ensure that it is suctioned to the underside of the filter element.

In a still further development, the nozzle comprises a nozzle extension that at least partially surrounds the tool orifice at a side opposite from the scraping blade. In an example, the tool is adapted to be arranged with a predefined scraping angle α relative to the horizontal slide surface. When the blade is in contact with the slide, a peripheral edge of the nozzle extension may be adapted to create a constant gap to the slide surface. In further examples, the peripheral edge of the nozzle extension has a predetermined profile, such that the gap varies along the circumferential direction.

The advantage of the nozzle extension is to optimize the airstream that is generated during suction, at the location of the scraping edge, thereby ensuring that all dissected material is suctioned into the body of the tool.

A dissection tool according to the invention can thus be used according to the inventive method to perform three functions: dissection, collection and transfer. The tool is further intended to be disposed of after use, which further reduces the risk of cross-contamination of the biological material.

In a third aspect, the invention relates to an apparatus for dissecting and transferring biological material from a sample disposed on a planar substrate, which is provided with a dissection tool as described above. The apparatus further comprises:
- a tool carrier having a mechanical interface for establishing an airtight connection with the corresponding mechanical interface at the second end of the dissection tool;
- a platform for supporting the planar substrate;
- a positioning system configured to move the tool carrier and the platform relative to each other and control their relative positions such that the scraping blade of the dissection tool selectively engages with the biological material in a predefined region of the sample;
- a vacuum generator or fitting for connection of an external vacuum generator;
- a pressure reservoir or fitting for connection of an external pressure reservoir; and
- a valve operable between a first position in which the internal passageway of the dissection tool is in communication with the vacuum generator and a second position in which the passageway is in communication with the pressure reservoir, for causing the collected biological material to be expelled into the collection tube.

In one example, the pressure reservoir is simply the atmosphere. In a further example the pressure reservoir is a pump. In a still further example, the pressure reservoir is a vessel containing pressurized air, held at pressure of 100 - 400 kPa.

Preferably, the apparatus comprises an imaging system for obtaining an image of the biological sample. Suitably, the imaging system is configured to identify the boundary between a region of interest containing biological material to be tested and an unwanted area containing material not to be tested. In other words, the imaging system identifies the shape of the region that is to be dissected. In some examples, the biological sample is a stained tissue sample and the imaging system simply recognizes the stained region. In other examples, the imaging system may be programmed with software to process a captured image of the tissue sample and identify the region of interest based on e.g. cell structure. The system may also be configured to identify the shape of the region to be dissected by comparing the captured image with a reference image that has been marked by a pathologist. Suitably, the imaging system is configured to transfer coordinates of the identified boundary to a controller of the positioning system.

The scraping blade of the dissection tool has a scraping edge of width w. As a result of the relative movement between the blade and the planar substrate in a direction of translation, the scraping edge cuts a track through the biological sample having a corresponding width. When the blade is formed by a section of a thin-walled tube, the width of the track depends on the outer diameter of the tube and the applied contact force.

During dissection, the dissection tool may be arranged such that the scraping blade is arranged at a scraping angle of between 30 and 60 degrees, although other angles may be desirable depending on the nature of the biological sample being dissected.

In a further development, the tool carrier is pivotably mounted to the apparatus to enable adjustment of the scraping angle and, if necessary, to bring the dissection tool to a vertical position, to facilitate automated coupling/uncoupling of the dissection tool and/ or automated attachment of the collection tube around e.g. the sealing collar of the tool.

Suitably, the positioning system comprises motorized actuators for relative movement in transverse X and Y directions and in the vertical direction Z. In some embodiments, the positioning system further comprises a rotation stage for adjusting a position of the platform (and biological sample) relative to the scraping blade about a rotation axis that is perpendicular to the planar substrate.

During dissection, the dissection tool is moved relative to the platform in a direction of translation T. In a further development, the tool carrier of the apparatus is rotational about an axis normal to the platform, to enable angular adjustment of the scraping edge relative to the direction of translation. This is advantageous as it allows the effective width of the scraping edge i.e. width measured perpendicular to the direction of translation, to be varied. Suitably, the positioning system comprises an actuator for controllable adjustment of angular orientation.

The actuators of the positioning system may be coupled to the platform, for moving the biological sample relative to the dissection tool and/or the actuators may be coupled to the tool carrier for moving the dissection tool relative to the platform.

In a further development, the apparatus is equipped with an air ionizer. Contact between the scraping edge and the planar surface on which the biological material is disposed produces friction, which can lead to a build-up of static charge on the biological material. The air ionizer conditions the air flowing around the dissection tool, and thus reduces any build-up of static charge that could adversely affect the collection of biological material during dissection.

In a still further development, the apparatus is equipped with a variable restrictor arranged in line between the vacuum generator and the filter element of the dissection tool, for regulating airflow through the tool nozzle. In use of the tool, the dissected material collected at the underside of the filter element will restrict the airflow through the filter, thereby increasing the restriction of the system as a whole. This will result in a loss of pressure and a reduction in airflow that could adversely affect the suction capability of the tool nozzle. Suitably, the apparatus is adapted to reduce the restriction of the variable restrictor in response to accumulation of dissected material at the underside of the filter, to enable a constant airflow through the internal passageway and the nozzle.

In one example, the apparatus is further equipped with a pressure sensor or flow sensor arranged in line between the vacuum generator and the filter element and the variable restrictor is controlled based on the measured pressure or measured flow. In a further example, the apparatus is configured to estimate an area of biological material that has been dissected by the scraping blade, and to reduce the restriction of the variable restrictor as a function of the dissected area. The imaging system may be configured to estimate the dissected area or it may be calculated based on the width of the scraping blade and the distance of each translation movement of the blade relative to the slide.

It will be appreciated by those skilled in the art that two or more of the above-mentioned embodiments, implementations, and/or aspects of the invention may be combined in any way deemed useful.

### BRIEF DESCRIPTION OF THE DRAWINGS

The invention will now be further elucidated with reference to the embodiments described hereinafter. In the drawings,
- Fig. 1a: schematically shows an example of an apparatus according to the invention, for dissection and transfer of biological material from a glass slide to a collection tube, the apparatus comprising an example of a dissection tool according to the invention;
- Fig. 1b: shows parts of the apparatus of Fig. 1a in a configuration in which material is removed from the slide and suctioned into the dissection tool;
- Fig. 1c: shows parts of the apparatus of Fig. 1a in a configuration in which the suctioned material is prepared for transfer to the collection tube;
- Fig. 1d: shows parts of the apparatus of Fig. 1a in a configuration in which the suctioned material is transferred to the collection tube.
- Fig. 2: is a side view of a further example of a dissection tool according to the invention.
- Fig. 3: is a side view of a still further example of a dissection tool according to the invention, with key internal features indicated via dotted lines.

It should be noted that items which have the same reference numbers in different figures, have the same structural features and the same functions, or are the same signals. Where the function and/or structure of such an item has been explained, there is no necessity for repeated explanation thereof in the detailed description.

### DETAILED DESCRIPTION OF EMBODIMENTS

Pathology diagnostic investigation of biological material, such as tissue and cells, forms the basis for many treatment decisions, particularly in oncology. For example, genomic-based tests are performed in order to inform therapy selection for individual patients diagnosed with cancer. The biological material/tissue may be obtained from a biopsy and is then, for example, embedded in paraffin and cut into thin slices which are fixed onto glass slides. These thin slices will be referred to as tissue samples. Other methods of obtaining and preparing biological material are known.

The tissue sample has a region of interest ROI containing material that is to be subjected to the diagnostic testing. The ROI can be identified by staining, or a pathologist may provide markings on a reference slide after analysis under a microscope. The ROI can also be identified via processing of a digital image of the sample. When the ROI has been identified, material is removed/dissected from the slide and then transferred to an analysis arrangement. Typically, the material is transferred to a collection tube, after which a sample preparation process steps, like cell lysis, purification and amplification, etc. and further necessary processing steps are performed. As will be understood, the reliability and efficiency of the analysis is optimized, by making sure that only dissected material from the ROI is present, but also by minimizing contamination.

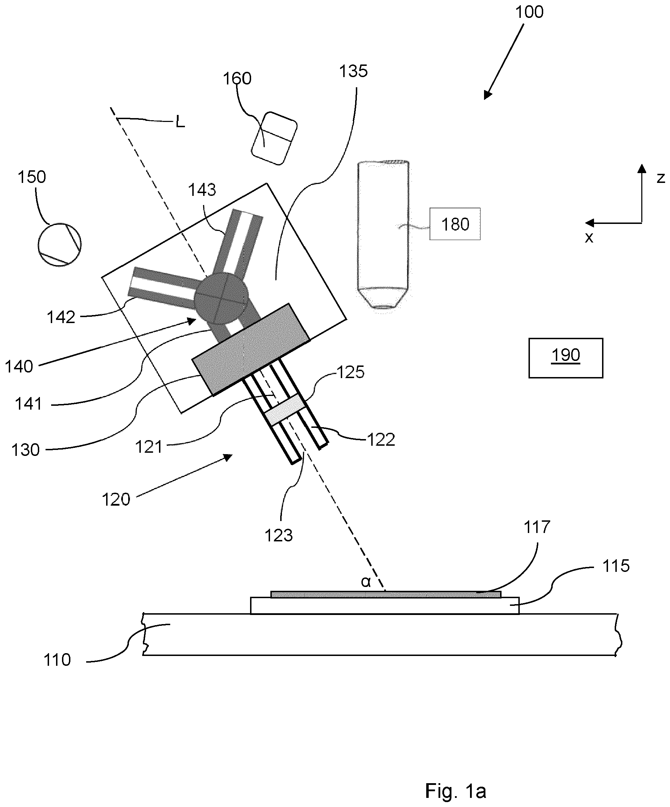

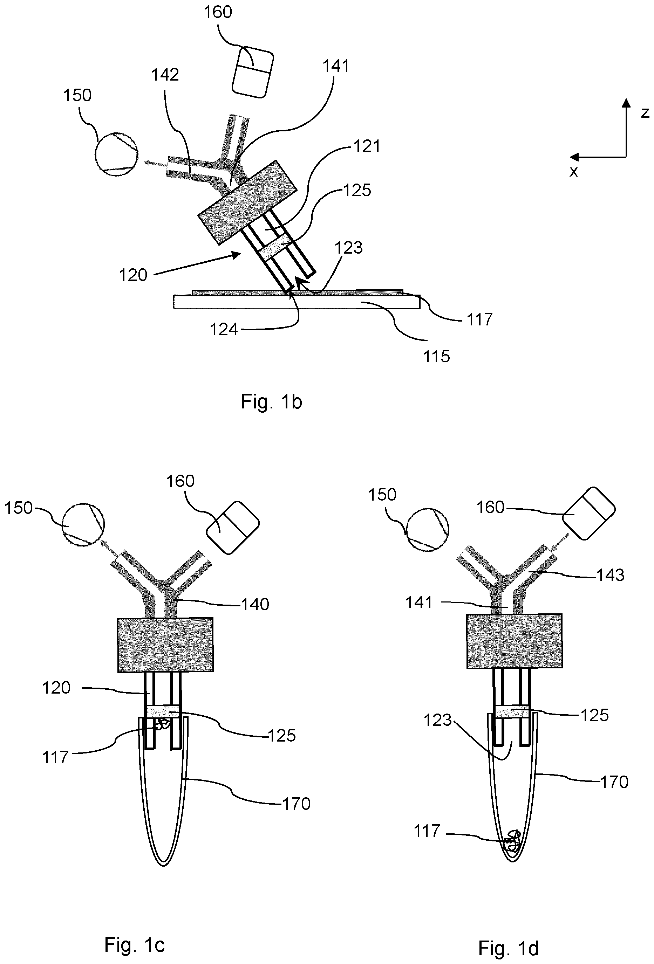

The present invention defines a method of dissecting biological material from a sample disposed on a planar substrate and transferring it to a collection tube in a manner that permits accurate material removal and minimizes contamination of the material to be analyzed. The method makes use of an inventive dissection tool that is selectively connectable to a source of underpressure and a source of overpressure. The dissection tool and apparatus of the invention will be described with reference to Fig. 1a.

Fig 1a schematically shows an example of an apparatus according to the invention. The apparatus 100 comprises a platform 110 for supporting a glass slide 115 on which a biological sample 117 is disposed, and a dissection tool 120 mounted to a tool carrier 130. The tool carrier is mounted to a positioning unit 135 that enables the position of the tool 120 to be adjusted relative to the platform 110. In the depicted example, the positioning unit 135 allows the tool 120 to be moved in vertical direction z and to be rotated about a vertical axis. The positioning unit forms part of a positioning system for enabling movement of the tool relative to the platform in translation directions X and Y. Preferably, the platform 110 is also movable and the positioning system comprises a controller 190 that controls relative movement such that the tool engages with the biological material 117 only in the region of interest. In the depicted example, the apparatus is further equipped with an imaging system 180 for identifying the location of the tissue and providing the ROI coordinates to the controller 190.

The dissection tool has an essentially hollow body that defines an internal passageway 121 extending between first and second ends of the tool body. An air-permeable filter element 125 is accommodated within the tool body and spans full diameter of the internal passageway. A first section of the internal passageway is defined between the filter element 125 and an orifice 123 of the tool at the tool first end. The portion of the tool body that surrounds the first section of the internal passageway is defined as a nozzle 122, which has a longitudinal centre axis L. The nozzle axis L may coincide the with the centre axis of the tool as a whole, as is the case in the example of Fig. 1a, although this is not necessary. The second end of the dissection tool comprises a mechanical interface for releasably connecting the tool to a mating mechanical interface on the tool carrier 130.

In the depicted example, the nozzle is formed by a thin-walled tube and a portion of an end face of the tube serves as a scraping blade 124 (refer Fig. 1b) that is used to mechanically detach biological material 117 disposed on the glass slide 115. The scraping blade may also be a separate part that extends from the nozzle end face at one side of the orifice 123.

During dissection, the tool is arranged such that the nozzle 122 is oriented at angle α relative to a horizontal surface of the platform 110, which also defines a scraping angle of the blade 124. The tool carrier 130 is pivotably mounted to the positioning unit 135, so as to enable adjustment of the angle α.

The apparatus is further equipped with a vacuum pump 150, a pressure reservoir 160 having a pressure of at least 100 kPa, a valve 140, and suitable tubing arranged such that the internal passageway 121 of the dissection tool 120 is selectively connectable to one of the pump 150 and the pressure reservoir 160. In the depicted example, the valve is a 3-way valve with a first port 141 in fluid communication with the mechanical interface on the tool carrier 130 and with passageway 121, a second port 142 in fluid communication with the vacuum pump 150 and a third port 143 in fluid communication with the pressure reservoir 160.

An embodiment of the method of the invention will now be described with reference to Figs. 1b - 1d, whereby for the sake of simplicity, only relevant features of the apparatus are depicted.

Fig. 1b shows the dissection tool in a material removal position and in a first step of the method, biological material 117 is scraped off the glass slide 115 and collected within the tool body. The tool 120 is arranged such that the scraping blade 124 engages with the slide surface at a scraping angle of e.g. 30 - 60 degrees. The tool is then moved relative to the slide in a translation direction x, such that a scraping edge of the blade, having a certain width in y-direction, cuts a track through the biological material 117. Suitably, the relative position of the scraping blade 124 and the slide 115 are controlled such that only material from the identified ROI is dissected via scraping.

Simultaneously with the step of scraping, an underpressure is generated at the second end of the dissection tool, which creates an uplifting airstream at the tool orifice 123, causing the dissected material to be suctioned into the passageway 121, where it is caught at an underside the filter element 125. During this step of suction, the valve 140 is in a first position in which the first and second ports 141, 142 are in fluid communication and the internal passageway 121 is in communication with the vacuum pump 150. The airstream holds the dissected material at the underside of the filter element 125 during the material removal process.

When dissection is complete, the position of the tool may be adjusted to a transfer position, in which the platform and dissection tool are distanced from each other. In a next step of the method, a collection tube 170 is attached at an outer surface of the dissection tool 120, as shown in Fig 1c, suitably in airtight manner. The valve 140 remains in the first position and the dissected material 117 is held at the underside of the filter element 125. As a result of the continued connection with the operating vacuum pump 150, air is evacuated from the collection tube 170.

A final step of the method comprises exposing the filter element 125 to an overpressure at the second end of tool 120. This is achieved by switching the valve 140 to a second position in which the first port 141 is in fluid communication with the third port 143 and with the pressure reservoir 160, as shown in Fig. Id. Preferably, the valve is switched to the second position when a vacuum situation is reached inside the tube 170, or a pressure essentially equal to the absolute pressure of the vacuum pump e.g. 1 - 10 kPa. Exposure to the overpressure generates a pressure pulse and an airstream, indicated by the arrow in Fig. Id, that flows towards the tool orifice 123. The aforementioned pressure pulse catapults the dissected material 117 from the underside of the filter element 125 into the collection tube 170.

The pressure reservoir 160 can simply be the atmosphere. Preferably, to generate a pulse of sufficient magnitude to ensure that all material is ejected from the filter element 125, a pump or a reservoir of pressurized air held within a cylinder is used to supply a pressure of 200 - 400 kPa. Preferably, the supplied pressure is lower than a clamping force generated by the airtight connection of the collection tube 170 around the outer surface of the tool body. This prevents the tube from being released by the pressure pulse, which would create a risk of losing the dissected material.

After exposure to a pressure greater than 100 kPa, the pressure in the collection tube is allowed to equilibrate with the atmosphere. The collection tube may then be detached from the dissection 120 and is suitably covered by an end cap.

The method of the invention therefore enables automated transfer of biological material from a sample disposed on a glass slide 115 into a collection tube 170, without the need to use any liquids during dissection or to "rinse" the dissected material out of the tool body. This not only simplifies the process, but also minimizes contamination.

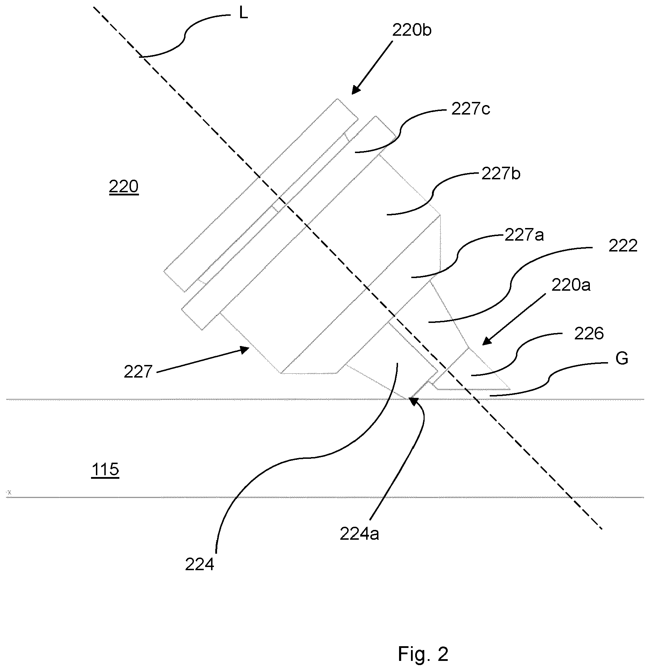

A further example of a dissection tool 220 according to the invention is shown in Fig. 2. The tool has a hollow tool body having an internal passageway in which the filter element is arranged. A first end 220a of the tool is provided with a nozzle 222, a portion of which serves as a scraping blade 224 having a scraping edge 224a that is brought into contact with a glass slide 115 and moved relative to the slide in order to dissect material. The scraping blade 224 in this example is a separate part formed from a thin-walled tube section made of a metal with a high yield stress. Remaining parts of the nozzle 222 and tool body may be formed from polymer material that is overmoulded to the scraping blade 224. Preferably, an internal surface of the scraping blade is provided with an anti-stick coating, to prevent sticking of dissected material during scraping and during transfer.

In use of the tool, a portion of the end face of the nozzle 222 that makes contact with the biological material on the slide 115 serves as the scraping blade 224. The scraping edge 224a in the depicted example is thus arcuate in shape and has a certain width that cuts a track through the biological material when the underside of the nozzle is brought into contact with the slide surface and is moved relative to the slide. The dissected material may therefore have a ribbon shape.

As explained, the underpressure generated by the vacuum pump creates an uplifting airstream that draws in the material dissected by the scraping edge 224a. To ensure that a ribbon of dissected material is suctioned into the tool body, it is important that the lifting effect of the airstream is optimized at the location of the scraping edge. In the depicted example, this is facilitated by an extension 226 of the nozzle 222 that surrounds approximately half of the tool orifice at a circumferential side opposite from the scraping edge 224a. The extension 226 forms a curved hood, whereby a maximum length of the extension 226, measured in longitudinal direction L relative to the scraping edge 224a, occurs at a circumferential location on the nozzle of 180 degrees relative the scaping edge. At either side of this 180° position, the length of the nozzle extension reduces.

In the depicted example, the dissection tool 220 is adapted to be mounted to a tool carrier, such that the scraping blade 224 is arranged at a scraping angle of 45 degrees relative to the horizontal surface of the slide. A blade axis of the scraping blade extends parallel to a centre axis L of the nozzle and tool, meaning that the scraping angle coincides with the angle of the tool axis relative to the surface of the slide.

The scraping edge 224a forms part of a suction nozzle. A peripheral edge of the extension 226 also forms part of the nozzle and thus shortens the gap G between the nozzle and the slide surface, thereby increasing the suction force exerted on the dissected biological material. The extension 226 is designed such that the gap G is substantially constant when the tool is arranged at 45 degrees to the slide surface and the scraping edge is in contact with the slide. In other examples, the nozzle extension is designed such that the gap varies along the circumference.

In addition to improving the lifting effect at the scraping edge 224a, the extension 226 can also serve as a physical barrier to prevent sideways deflection of a dissected ribbon of material and retain it within the nozzle.

At a second end of the tool 220b, a mechanical interface is provided for releasably attaching the tool 220 at a mating interface on the tool carrier. Suitably, the interface permits an airtight connection to ensure optimal suction through the tool body and a high degree of mechanical alignment, to promote accurate positioning of the scraping edge 224a relative to the slide 115. A click-type fitting, clamping or a bayonet attachment is advantageous for the purposes of speed and automation, but other types of interface, such as threaded connection, are also possible.

The dissection tool 220 further comprises an external connection interface for receiving the collection tube that is placed over the tool body. In the depicted example, the outside of the tool body is fitted with a collar 227 made of an elastomeric sealing material for establishing an airtight connection with the collection tube. The dimensions of the collar thus depend on the collection tube used. Preferably, the collar has a chamfered edge 227a for guiding the open end of the collection tube over a main portion 227b of the collar, which has an external diameter that will seal against the internal diameter or rim of the collection tube. Suitably, the collar also has a larger-diameter flange portion 227c that will retain a rim of the collection tube, to prevent damage that might otherwise occur when the tube is attached and evacuated.

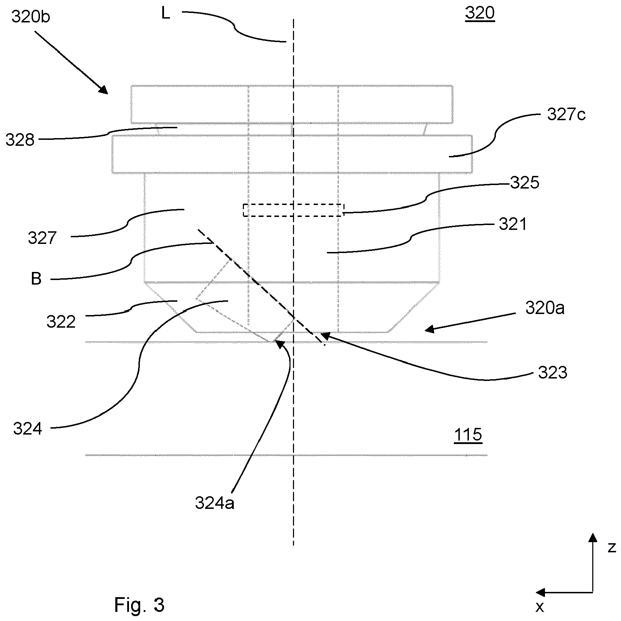

Fig. 3 shows a further example of a dissection tool according to the invention. The tool 320 in this example is adapted to be arranged such the longitudinal centre axis L of the nozzle 322 is perpendicular to the glass slide 115 during dissection. At the tool first end 320a, an end face of the nozzle 322 surrounds the orifice 323 at the entrance to the internal passageway 321, indicated via dotted lines. The tool comprises a metal scraping blade 324 embedded in the nozzle, whereby part of the blade protrudes from the end face of the nozzle. The embedded part of the blade is indicated via dotted lines. In the depicted example, the scraping blade if formed from a thin piece of metal that is bent into semi-cylindrical shape around a blade axis B. The scraping blade may also be a straight, flat part that resembles a chisel.

The blade axis B extends at an angle relative to the nozzle axis L, and the scraping angle is defined by the angle between the blade axis B and the slide surface. The scraping angle can, of course, be varied by adjusting the orientation of the tool body. Suitably, the scraping blade 324 extends towards the nozzle axis L, such that the scraping edge 324a of the blade is located below the tool orifice 323.

In the depicted example, the tool 320 comprises a container part 328. The internal passageway 321 extends through this part 328 and the filter element 325 (indicated via dotted lines) is arranged in the passageway. The mechanical interface for releasably connecting the tool to the carrier is provided at the tool second end 320b, at the second end of the container part 328.

The nozzle 322 is formed from polymer material that is overmoulded to the container part 328. The scraping blade 324 is embedded in the end face of the nozzle. It is also possible for the nozzle and container part to be entirely formed from material that is overmoulded to the metal scraping blade 324.

In the depicted example, the overmoulded portion not only comprises the nozzle 322, but further comprises the second mechanical interface 327 or collar for connection of the collection tube. Like the example shown in Fig. 2, the collar 327 has a flange part 327c for retaining the rim of the collection tube.

After use, a dissection tool according to the invention is disposed of and a new tool is connected to the apparatus, thereby eliminating the risk of cross-contamination.

Examples, embodiments or optional features, whether indicated as non-limiting or not, are not to be understood as limiting the invention as claimed. It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

### Reference numerals

- 100: dissection apparatus
- 110: platform
- 115: slide
- 117: tissue sample
- 120, 220, 320: dissection tool
- 121, 321: internal passageway through dissection tool
- 122, 222, 322: nozzle
- 123, 323: tool orifice
- 124, 224, 324: scraping blade of dissection tool
- 125, 325: air-permeable filter element
- 130: tool carrier
- 135: positioning unit
- 140: 3-way valve
- 141, 142, 143 1^{st}, 2^{nd}, 3^{rd}: valve ports
- 150: vacuum pump
- 160: pressure reservoir
- 170: collection tube
- 180: imaging system
- 190: controller of positioning system

- 220a, 320a: 1^{st} end of dissection tool
- 220b, 320b: 2^{nd} end of dissection tool
- 224a, 324a: scraping edge of scraping blade
- 226: extension of nozzle
- 227, 327: sealing collar
- 227a: chamfered edge of sealing collar
- 227b: main body of sealing collar
- 227c, 327c: retaining flange of sealing collar
- L: longitudinal axis of tool nozzle
- B: blade axis
- α: scraping angle of blade relative to slide surface
- G: gap between nozzle and slide surface

## Claims

1. A method of transferring biological material (117) from a sample disposed on a planar substrate (115) into a collection tube (170), using a dissection tool (120, 220, 320) that has an internal passageway (121, 321) extending between first and second ends (220a, 220b; 320a, 320b) of the tool and an air-permeable filter element (125, 325) that spans the internal passageway, whereby the tool first end is provided with an orifice (123, 323) and a scraping blade (124, 224, 324) at one side of the orifice, the method comprising steps of:
a) physically detaching the biological material from the planar substrate (115) using the scraping blade (124, 224, 324);
b) during step a), generating an underpressure at the tool second end (220b, 320b), to create an uplifting airflow at the tool orifice (123, 323), which suctions detached material into the internal passageway (121, 321), where it is held at an underside of the filter element (125, 325);
c) arranging the collection tube around the orifice, so as to be in sealing contact with a connection interface (227, 327) on the dissection tool, and
d) generating an overpressure at the tool second end, so as to create an airstream and pressure pulse that ejects the biological material held at the underside of the filter element (125, 325), transferring the material (117) into the container tube.

2. The method of claim 1, wherein:
• the step of generating an underpressure comprises connecting the tool internal passageway (121, 321) to a vacuum generator (150);
• the step of generating an overpressure comprises connecting the internal passageway to a pressure reservoir (160); and
• the method comprises a further step of using the vacuum generator to evacuate the collection tube (170) before the internal passageway is connected to the pressure reservoir.

3. The method of claim 1 or 2, wherein step a) comprises bringing the scraping blade (124, 224, 324) into contact with the planar substrate (115) and moving the dissection tool relative thereto and wherein the method further comprises controlling the relative positions of the dissection tool (120, 220, 320) and the substrate such that the scraping blade engages with the biological material only in a region of interest.

4. The method of any preceding claim, wherein step b) comprises controlling an airflow through the dissection tool by changing the restriction of a variable restrictor in response to one or more of:
• a pressure or airflow, measured downstream from the filter element (125, 325) of the dissection tool;
• an estimated area of biological material that has been detached from the substrate during step a).

5. Dissection tool (120, 220, 320) for removing and collecting biological material (117) from a sample disposed on a planar substrate (115), the tool comprising first and second ends (220a, 220b; 320a, 320b), an internal passageway (121, 321) extending therebetween, and an orifice (123, 323) at the tool first end, wherein the tool further comprises:
• an air-permeable filter element (125, 325) arranged within the internal passageway (121) to span the full diameter thereof, whereby a first section of the internal passageway is defined between the tool orifice (123) and a first side of the filter element that faces the orifice;
• a nozzle (122, 222, 322) that surrounds the first section of the internal passageway;
• a scraping blade (124, 224, 324) arranged at one side of the tool orifice (123, 323);
• a first connection interface, provided at the tool second end, for releasably connecting the dissection tool in an airtight manner to a mating interface on a tool carrier (130); and
• a second connection interface (227, 327) for establishing an airtight connection with a collection tube (170).

6. Dissection tool according to claim 5, wherein the scraping blade (124, 224, 324) is formed from a thin-walled section of a tube, whereby an end face of the tube section serves as a scraping edge (224a, 324a) of the scraping blade.

7. Dissection tool according to claim 5, wherein the blade has a flat scraping edge (224a, 324a).

8. Dissection tool according to any of claims 5 - 7, wherein the scraping blade (324) extends from an end face of the nozzle (322).

9. Dissection tool according to claim 6, wherein the scraping blade (124, 224) forms part of the nozzle (122, 222).

10. Dissection tool according to claim 8, wherein the scraping blade (324) extends at an angle relative to a longitudinal axis L of the nozzle (322).

11. Dissection tool according to any of claims 5 - 10, wherein the second connection interface is formed by a flanged collar (227, 327) and is one of:
• a separate part made of elastomeric material; or
• is an integral part of the tool body.

12. Dissection tool according to any of claims 5 - 11, wherein:
• the scraping blade (124, 224, 324) is a separate part made of metal; and
• the nozzle (122, 222, 322) or an entire body of the tool is made of a polymer material.

13. Dissection tool according to any of claims 5 - 12, wherein the nozzle (122, 222, 322) comprises a nozzle extension (226) at an opposite circumferential side from the scraping blade (124, 224, 324) and wherein a peripheral edge of the nozzle extension creates a predefined gap G to the planar substrate (115) when the scraping blade is in contact with the planar substrate at a predefined scraping angle.

14. Apparatus for dissecting and transferring biological material from a sample disposed on a planar substrate, comprising a dissection tool (120, 220, 320) according to one of claims 5 - 13 and further comprising:
• a tool carrier (130) having a mechanical interface for an airtight connection of the tool second end (220b);
• a platform (110) for supporting the planar substrate (115);
• a positioning system (135) configured to move the tool carrier (130) and the platform (110) relative to each other and control their relative positions such that the scraping blade (124, 224, 324) of the dissection tool selectively engages with the biological material (117) in a predefined region thereof;
• a vacuum generator (150) or a fitting for connection of an external vacuum generator;
• a pressure reservoir (160) or a fitting for connection of an external pressure reservoir;
• a valve (140) operable between a first position in which the internal passageway (121, 321) of the dissection tool is in communication with the vacuum generator (150) and a second position in which the passageway is in communication with the pressure reservoir (160), for causing the collected biological material to be expelled into a collection tube.

15. Apparatus according to claim 14, further equipped with a variable restrictor arranged in line between the vacuum generator (160) and the filter element (125, 325) of the dissection tool and means for controlling the restriction of the variable restrictor, and one or more of:
• a flow sensor or a pressure sensor arranged in line between the vacuum generator (150) and the filter element (125, 325);
• means for estimating an area of biological material dissected from a sample during use of the tool;
wherein the means for controlling is configured to adjust the restriction of the variable restrictor based on the measured flow or the measured pressure or the estimated area of dissected biological material.
